Europäisches Patentamt

European Patent Office

Office européen des brevets

(19)

(11) Veröffentlichungsnummer: **0 015 443**
**B1**

(12) **EUROPÄISCHE PATENTSCHRIFT**

(45) Veröffentlichungstag der Patentschrift:
02.02.83

(21) Anmeldenummer: 80100851.7

(22) Anmeldetag: 21.02.80

(51) Int. Cl.³: **A 61 M 5/14**, A 61 M 25/00,
F 16 K 7/16

(54) **Medizinisches Gerät zur Zuführung oder Entnahme von Flüssigkeit mit Hilfe einer Injektionsspritze.**

(30) Priorität: 24.02.79 DE 7905206 U

(43) Veröffentlichungstag der Anmeldung:
17.09.80 Patentblatt 80/19

(45) Bekanntmachung des Hinweises auf die Patenterteilung:
02.02.83 Patentblatt 83/5

(84) Benannte Vertragsstaaten:
AT BE CH DE FR GB IT LU NL SE

(56) Entgegenhaltungen:
DE-A-1 550 614
DE-A-2 601 993
DE-A-2 652 170
DE-A-2 714 557
DE-A-2 819 900
DE-A-2 830 800
DE-B-2 029 179
DE-C-1 216 489
DE-U-7 727 563
DE-U-7 808 708
DE-U-7 812 248
DE-U-7 905 206

(73) Patentinhaber: Intermedicat GmbH, Gerliswilstrasse 45,
CH-6020 Emmenbrücke (CH)

(72) Erfinder: Werner, Hans-Theo, Am Steig 2,
Edermünde-Grifte (DE)
Erfinder: Herlitze, Gerhard, Baunatal-Guntershausen
(DE)

(74) Vertreter: von Kreisler, Alek, Dipl.-Chem. et al,
Deichmannhaus am Hauptbahnhof, D-5000 Köln 1 (DE)

## Medizinisches Gerät zur Zuführung oder Entnahme von Flüssigkeiten mit Hilfe einer Injektionsspritze

Die Erfindung bezieht sich auf ein medizinisches Gerät zur Zuführung oder Entnahme von Flüssigkeiten mit Hilfe einer Injektionsspritze, das ein Gehäuse mit einer zylindrischen Wand aufweist, in die eine Klemmhülse eingepasst ist, deren Durchgang als Einsducköffnung für den Kegelansatz der Injektionsspritze dient und deren ebene Stirnfläche den Randteil eines als elastische Platte mit selbstschliessender Flüssigkeitsdurchlassöffnung ausgebildeten Ventilkörpers gegen eine Ringschulter des Gehäuses am Übergang zu einem Hauptdurchströmungskanal andrückt.

Ein solches Gerät ist bei Ansatzstücken für Venenverweilkanülen und Venenkatheter bekannt (DE-U Nr. 7808708 und DE-A Nr. 2714557). Dabei ist die Flüssigkeitsdurchlassöffnung der in dem Gehäuse festgeklemmten gummielastischen Platte als von dem Kegelansatz einer Injektionsspritze mechanisch aufweitbarer Durchgang ausgebildet, der sich nach Zurückziehen des Kegelansatzes selbsttätig schliesst. Bei diesem Öffnungsvorgang durch radiale Zugspannungen in der Platte, die mittels des Spritzenkegels durch axiale Verschiebung hervorgerufen werden, steigt die Kraft zum Öffnen des Ventils sofort progressiv an. Dies ist darauf zurückzuführen, dass die Platte eine erhebliche Wandstärke aufweisen muss, damit die Eigenelastizität des Materials ausreicht, um die Schlitze nach Herausziehen des Spritzenkegels durch abdichtende gegenseitige Anlage ihrer Ränder zu schliessen bzw. geschlossen zu halten. Zur Erzielung der abgedichteten Schliessstellung muss die Platte daher bereits eine Vorspannung haben, deren effektive Kraft sich progressiv zum axialen Hub ändert, so dass beim Einsetzen des Spritzenkegels zum Öffnen des Ventils die notwendige Kraft in sehr starkem Masse wegeabhängig ansteigt. Hierdurch wird die Handhabung des Gerätes erschwert und beispielsweise bei einem Ansatzstück eines Venenverweilkatheters, dessen Kapillarschlauch in das Blutgefäss eines Patienten eingesetzt ist, kann das schwergängige Öffnen der Flüssigkeitsdurchlassöffnung zu unerwünschten Schmerzbelastungen des Patienten durch Flächenpressung führen. Da einem Patienten aus einer Füllung der Injektionsspritze in zeitlichen Abständen auch mehrfach geringe Menge zugegeben werden, können je nach Grösse der Öffnungskräfte erhebliche Mehrbelastungen für den Patienten auftreten. Die Öffnungsstellung der Flüssigkeitsdurchlassöffnung ist von aussen nicht erkennbar und es bedarf deshalb zur Feststellung des Öffnungsgrades des Ventils besonderer Feinfühligkeit des Arztes. Es kann passieren, dass injiziert wird bevor das Ventil voll geöffnet ist. Merkt der Arzt dies und erhöht zur Erreichung der notwendigen Injektionsmittelrate den Spritzendruck, so kann sich eine Venenwandläsion durch zu hohe Ausfliessgeschwindigkeit und damit verbundener Jetstrahlbildung ergeben, die für den Patienten schädlich ist.

Auf dem Sektor der Kraftfahrzeugtechnik ist ein Schliessorgan für pneumatische Rückschlagventile zur Trennung der Frischluftleitung und der Auspuffkammern eines Kraftfahrzeuges bekannt (DE-A Nr. 1550614). Dieses Schliessorgan besteht aus einem Ventilteller, aus einem diesem gegenüber in Strömungsrichtung versetzten Dichtring sowie aus mehreren den Ring und den Ventilteller verbindenden kegelig oder zylindrisch angeordneten Knickfedern bildenden Stegen. Der Ventilteller wird durch kontinuierlich geförderte Frischluft von seinem Sitz weggedrückt, wobei die Stege einknicken. Da die Federkraft von Knickfedern mit zunehmender Knickung abnimmt, wird zum Niederhalten des Ventiltellers in Öffnungsstellung gegenüber der anfänglichen Öffnungsenergie eine wesentlich verminderte Luftenergie benötigt, was eine Senkung der Drosselwirkung des Rückschlagventils zur Folge hat. Ein solches Schliessorgan ist zum Einbau in medizinisches Gerät zur Zuführung oder Entnahme von Flüssigkeiten mit Hilfe einer Injektionsspritze nicht geeignet, weil der erwähnte degressive wegeabhängige Kraftverlauf ungünstig ist.

Bei einem medizinischen Gerät, das die Zuspritzung von Flüssigkeiten in ein Blutgefäss eines Patienten ermöglicht, sollte das Ventil so ausgebildet sein, dass beim Zuspritzen ein Druckpunkt vorhanden ist, der dem Anwender exakt anzeigt, dass bei einem bestimmten Kraftaufwand Flüssigkeit injiziert wird. Bei langsamem Zuspritzen soll im praktischen Toleranzbereich der Zuspritzgeschwindigkeit kein erhöhter Kraftaufwand zum Öffnen des Ventils spürbar sein.

Um den Patienten vor hoher medikamentöser Belastung zu schützen und zur Verhinderung einer Venenwandläsion durch zu hohe Ausfliessgeschwindigkeit und damit verbundener Jetstrahlbildung, ist es erforderlich, dass die Ventilöffnungskraft bei Zuspritzgeschwindigkeiten über den gewünschten Toleranzbereich hinaus spürbar progressiv ansteigt. Die Knickfedern des Schliessorgans für pneumatische Rückschlagventile erfüllen diese Forderungen nicht, weil die Federkraft bei zunehmender Öffnung des Ventils abnimmt. Ausserdem wird durch die kegelig oder zylindrisch angeordneten Stege die Höhe des Ventilkörpers in einem Masse vergrössert, das ihn ebenfalls für medizinische Geräte zur Zuführung oder Entnahme von Flüssigkeiten mit Hilfe einer Injektionsspritze untauglich macht, die bekanntlich klein sind.

Der Erfindung liegt die Aufgabe zugrunde, ein medizinisches Gerät der eingangs erwähnten Art dahingehend zu verbessern, dass die vom Anwender aufzubringende Kraft sehr gering ist, um das Ventil zu öffnen und spürbar progressiv zunimmt, wenn die Zuspritzgeschwindigkeit einen für den Patienten verträglichen Toleranzbereich überschreitet.

Diese Aufgabe wird dadurch gelöst, dass mit dem Randteil der Platte über elastische radiale Stege ein Mittelteil der Platte verbunden ist, der in Einbaulage der Platte in der Ebene des Randteiles gegen die Stirnfläche der Klemmhülse abdichtend anliegt.

Auf diese Weise erhält der Ventilkörper den erwähnten ‚Druckpunkt', der dem Anwender exakt anzeigt, dass bei einem bestimmten Kraftaufwand das Arzneimittel injiziert wird. Bei langsamen Zuspritzen ist kein erhöhter Kraftaufwand zum Wegdrücken des Mittelteiles von dem stirnseitigen Rand der Klemmhülse spürbar. Erst wenn die Zuspritzgeschwindigkeit unerwünscht zunimmt, steigt die Ventilöffnungskraft spürbar progressiv, so dass der Patient vor hoher medikamentöser Belastung geschützt und eine Venenwandläsion durch zu hohe Ausfliessgeschwindigkeit und damit verbundener Jetstrahlbildung verhindert wird. Diese vorteilhafte Funktion ergibt sich dadurch, dass die elastischen Stege so angeordnet sind, dass sich in Einbaulage der Platte zunächst Druckspannungen in den Stegen aufbauen, die den Mittelteil dichtend gegen den stirnseitigen Rand der Klemmhülse drücken. Die Überwindung der sich aus den Druckspannungen ergebenden Kraft zum Öffnen des Ventils entspricht dem gewünschten Druckpunkt. Der weitere wegeabhängige Kraftanstieg ist zunächst sehr gering und praktisch vernachlässigbar, während über diesen Bereich hinaus bei zunehmendem Ventilöffnungsweg, hervorgerufen durch dynamische Kraft bei hoher Zuspritzgeschwindigkeit, der Kraftanstieg progressiv verläuft. Dies ist darauf zurückzuführen, dass die in den Stegen zunächst vorhandenen Druckspannungen in Zugspannungen übergehen und die Stege in diesem Übergangsbereich fast spannungsneutral sind, um dann im weiteren Verlauf mit zunehmender Zugspannung den typischen progressiven Kraftverlauf von Elastomeren aufzuweisen. Ausserdem hat die Anordnung des Randbereiches, der Stege und des Mittelteiles der Platte in einer gemeinsamen Ebene den Vorteil einer einem kleinen medizinischen Gerät angemessenen geringen Höhe.

In vorteilhafter Ausgestaltung der Erfindung ist vorgesehen, dass auf der Oberseite des Mittelteiles mindestens ein hochstehender rippenartiger Vorsprung oder eine Vertiefung in Form einer Nut ausgebildet ist. Hierdurch wird bewirkt, dass die Öffnung des Kegelansatzes einer Injektionsspritze immer in gewissem Abstand zur Oberfläche des Mittelteiles der Platte gehalten wird, so dass bei der Entnahme von Flüssigkeiten die Öffnung der Injektionsspritze sich nicht an der Oberfläche des Mittelteiles der Platte festsaugen und verschlossen werden kann.

Ferner ist es zweckmässig, dass an der Stirnfläche der Klemmhülse und/oder an der Ringschulter des Gehäuses umlaufende Halterippen und -nuten für die Platte ausgebildet sind. Diese verstärken die von der Klemmhülse gegen den Rand der Platte ausgeübte Haltekraft.

Die Klemmhülse kann mit einer Kappe verbunden sein, an der ein Klappdeckel befestigt ist, und die gegen axiales Abziehen von der zylindrischen Wand des Gehäuses gesichert ist. Der Klappdeckel verschliesst die Einsecköffnung für den Kegelansatz der Injektionsspritze, so dass nach dem Zuspritzen keine Luftkeime in die Zuspritzöffnung gelangen können. Als Sicherung gegen axiales Abziehen der Kappe können zusammengreifende Rippen oder Vorsprünge und Vertiefungen auf der Aussenfläche der Wand des Gehäuses und der Innenfläche der Kappe dienen.

Vorteilhafterweise ist der Randteil der Platte gegen den Hauptdurchströmungskanal des Gehäuses gerichtet. Hierdurch wird verhindert, dass der von dem Kegelansatz der Injektionsspritze weggedrückte Mittelteil der Platte so weit in den lichten Querschnitt des Hauptdurchströmungskanals hineinragt, dass er die Strömung in dem Hauptdurchströmungskanal beeinflusst.

In der Zeichnung sind Ausführungsbeispiele der Erfindung schematisch dargestellt. Es zeigt:

Fig. 1    einen Schnitt durch ein medizinisches Gerät mit einem Ventilkörper in Form einer Platte in geschlossenem Zustand,

Fig. 2    die Anordnung nach Fig. 1 in geöffnetem Zustand mit eingestecktem Kegelansatz einer Injektionsspritze,

Fig. 3    einen Schnitt durch das mittels der Platte verschlossene Einlassende des Hauptdurchströmungskanals, und

Fig. 4    einen Schnitt A-B durch die Anordnung nach Fig. 3 ohne Klemmhülse.

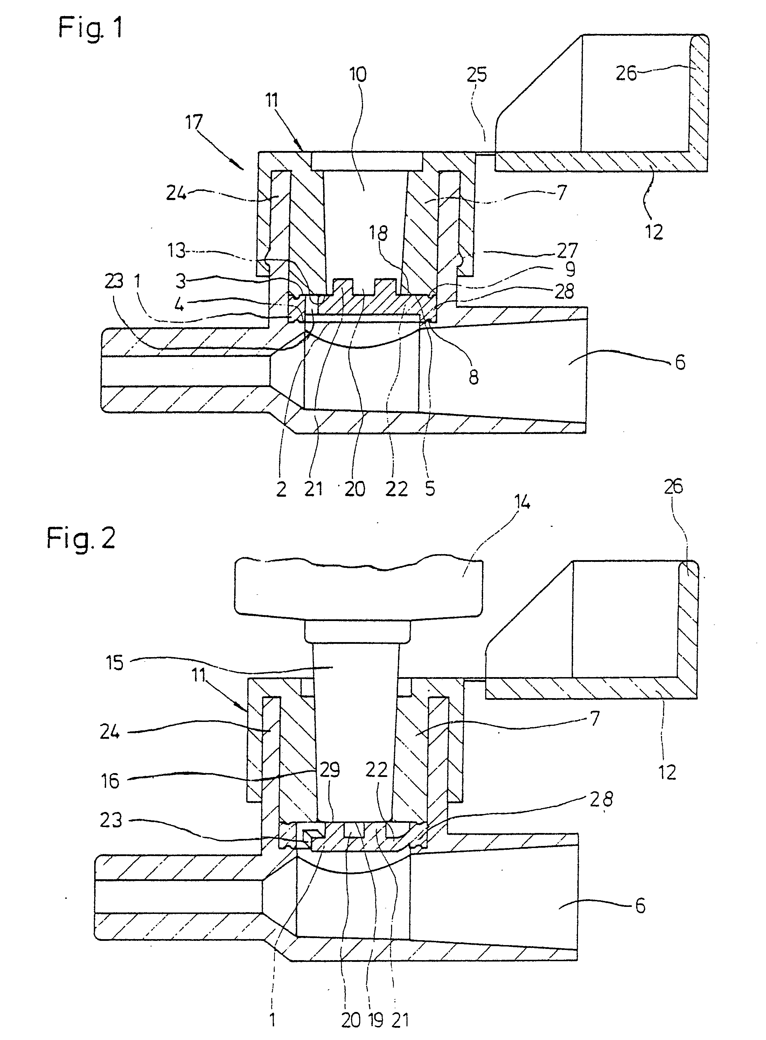

Der in den Fig. 1, 2 und 4 dargestellte Ventilkörper ist als elastische Platte mit nach unten gerichtetem Randteil 28 ausgebildet. Die Platte 1 ist mit den Stirnflächen 2 und 3 ihres Randteiles 28 fest zwischen der Ringschulter 4 an der Auslassöffnung des Gehäuses 17 zum Hauptdurchströmungskanal 6 und der Stirnfläche 5 einer Klemmhülse 7 eingespannt. Als zusätzlicher Halt sind umlaufende Halterippen bzw. -nuten 8 und 9 wirksam.

Die Klemmhülse 7 ist mit einer Kappe 11 ausgestattet, die auf eine zylindrische Wand 24 des Gehäuses 17 aufgesteckt ist. Der Durchgang 10 der Klemmhülse 7 verjüngt sich nach unten und dient als Einstecköffnung, die einen dichtenden Sitz des Kegelansatzes 15 einer Injektionsspritze 14 ermöglicht.

Die Einstecköffnung kann mittels eines Klappdeckels 12 verschlossen werden, der mit einer Lasche 25 an der Kappe 11 befestigt ist und mit einem umlaufenden Rand 26 die Kappe 11 umschliesst.

Als Sicherung gegen ein unerwünschtes Abziehen der Kappe 11 von der Wand 24 dienen zusammengreifende, durchgehende oder unterbrochene umlaufende Rippen 27 an der Kappe 11 und an der Wand 24.

Die Platte 1 aus elastischem Material weist mehrere Umfangsschlitze 23 auf, die durch radiale Stege 22 zwischen dem nach unten gerichteten Randteil 28 und einem als Dichtplatte wirkenden Mittelteil 21 voneinander getrennt sind. Die Stirnfläche 5 der Klemmhülse 7 verschliesst gemäss Fig. 1 die Umfangsschlitze 23 bei unbelastetem Mittelteil 21. Auf der Oberseite des Mittelteiles 21 befindet sich eine Nut 20.

Die Platte 1 dichtet mit ihrer Dichtfläche am Mittelteil 21 die Einstecköffnung am Dichtrand 14 der Klemmhülse 7 gegen den Hauptdurchströmungskanal 6 ab.

Die Stirnseite 19 des in den Durchgang 10 eingeführten Kegelansatzes 15 der Injektionsspritze 14 drückt auf die Oberfläche 29 des Mittelteiles 21, wodurch der an den elastischen Stegen 22 aufgehängte Mittelteil 21 nach unten gedrückt wird und den Zugang zum Hauptdurchströmungskanal 6 freigibt. Durch die zu grossen Durchbrüchen erweiterten Umfangsschlitze 23, die von den Stegen 22 begrenzt werden, kann das zugespritze Medium einströmen.

Die auf die Injektionsspritze 14 wirkenden Ventil- und Systemdruckkräfte werden durch Friktion zwischen dem Kegelmantel 16 des Kegelansatzes 15 und der kegelförmigen Einstecköffnung in der Klemmhülse 7 aufgenommen. Bei Entfernen der Injektionsspritze 14 schliesst sich der Zugang zum Hauptdurchströmungskanal 6 durch Gegeneinanderlegen des Dichtrandes 13 der Klemmhülse 7 und der Dichtfläche 18 durch Zurückschwingen des Mittelteiles 21 im gleichen Masse, wie der Kegelansatz 15 aus der Platte 1 herausgezogen wird.

Die Platte 1 kann in ihren elastischen Stegen 22 zum Mittelteil 21 dünn ausgebildet sein, so dass sie sich mittels des in die Einstecköffnung eingeführten Kegelansatzes 15 der Injektionsspritze 14 leicht und praktisch ohne Kraftaufwand aufdrükken lässt. Der Mittelteil 21 weicht aufgrund seiner elastischen Aufhängung bzw. Konstruktion bereits bei leichtem Andruck des Kegelansatzes 15 aus und ermöglicht das Zuspritzen von Flüssigkeit. Die Eigenelastizität der eingespannten Platte 1 erlaubt ein beliebig häufiges Zuspritzen von Infusionslösung oder dergleichen bei stets gleichbleibenden Dichtwirkung der Platte 1. Beim Zurückziehen und Entfernen der Injektionsspritze 14 bleibt praktisch keine Restmenge von Injektionsflüssigkeit zwischen dem Kegelansatz 15 und der Platte 1 zurück, wodurch die Gefahr einer Zuführung kontaminierter Injektionsflüssigkeit zum Patienten vermieden ist. Die Klemmhülse 7 gestattet eine Herausnahme der Platte 1 aus dem Gehäuse 17 zum Auswechseln gegen eine gleiche neue Platte 1 oder gegen eine Platte 1 mit abgewandeltem Mittelteil 21 zur Erzielung einer geänderten Durchflussöffnung.

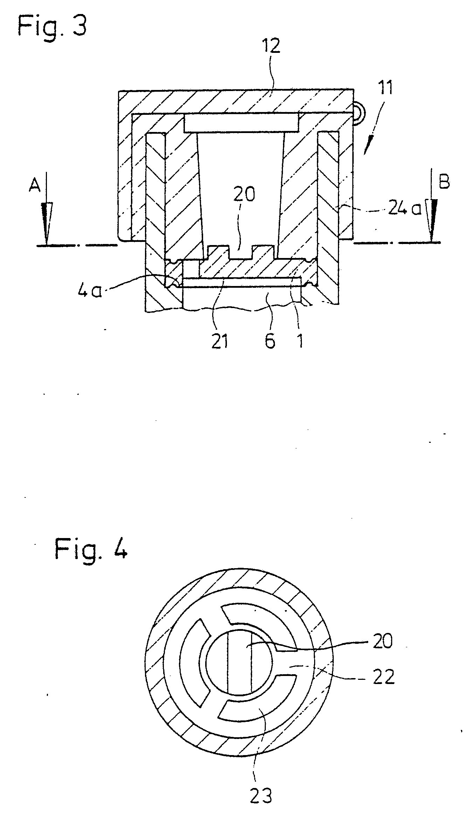

Gemäss Fig. 3 kann die Platte 1 in Verbindung mit der Klemmhülse und der Kappe 11 zum Verschluss der Axialöffnung am Einlassende des Hauptdurchströmungskanals 6 verwendet werden. Die Axialöffnung ist in diesem Falle entsprechend dem Gehäuse 17 mit einer zylindrischen Wand 24a und mit einer Ringschulter 4a versehen.

## Patentansprüche

1. Medizinisches Gerät zur Zuführung oder Entnahme von Flüssigkeiten mit Hilfe einer Injektionsspritze, das ein Gehäuse (17) mit einer zylindrischen Wand (24) aufweist, in die eine Klemmhülse (7) eingepasst ist, deren Durchgang (10) als Einstecköffnung für den Kegelansatz (15) der Injektionsspritze (14) dient, und deren ebene Stirnfläche (5) den Randteil (28) eines als elastische Platte (1) mit selbstschliessender Flüssigkeitsdurchlassöffnung ausgebildeten Ventilkörpers gegen eine Ringschulter (4) des Gehäuses (17) am Übergang zu einem Hauptdurchströmungskanal (6) andrückt, dadurch gekennzeichnet, dass mit dem Randteil (28) der Platte (1) über elastische radiale Stege (22) ein Mittelteil (21) der Platte (1) verbunden ist, der in Einbaulage der Platte (1) in der Ebene des Randteiles (28) gegen die Stirnfläche (5) der Klemmhülse (7) abdichtend anliegt.

2. Gerät nach Anspruch 1, dadurch gekennzeichnet, dass auf der Oberseite des Mittelteiles (21) mindestens ein hochstehender rippenartiger Vorsprung angeordnet ist.

3. Gerät nach Anspruch 1, dadurch gekennzeichnet, dass auf der Oberseite des Mittelteiles (21) mindestens eine Vertiefung in Form einer Nut (20) ausgebildet ist.

4. Gerät nach einem der Ansprüche 1 bis 3, dadurch gekennzeichnet, dass der Randteil (28) der Platte (1) gegen den Hauptdurchströmungskanal (6) des Gehäuses (17) gerichtet ist.

5. Gerät nach einem der Ansprüche 1 bis 4, dadurch gekennzeichnet, dass an der Stirnfläche (5) der Klemmhülse (7) und/oder an der Ringschulter (4) des Gehäuses (17) umlaufende Halterippen und -nuten (8, 9) für die Platte (1) ausgebildet sind.

6. Gerät nach einem der Ansprüche 1 bis 5, dadurch gekennzeichnet, dass die Klemmhülse (7) mit einer Kappe (11) verbunden ist, an der ein Klappdeckel (12) befestigt ist, und die gegen axiales Abziehen von der zylindrischen Wand (24) des Gehäuses (17) gesichert ist.

## Claims

1. Medical apparatus for the introduction or removal of liquids by means of a syringe comprising a housing (17) having a cylindrical wall (24) into which a clamping sleeve (7) is fitted whose passage (10) serves as an insert opening for the cone portion (15) of the syringe (14) and whose plan end surface (5) presses the edge portion (28) of a valve body designed as an elastic plate (1) having a self-closing liquid passage opening against an annular shoulder (4) of the housing (17) at the transition to a main flow channel (6), characterized in that there is connected to the edge portion (28) of the plate (1) via resilient radial webs (22) a central part (21) of the plate (1) which in mounting position of the plate (1) sealingly adjoins in the plane of the edge portion (28) the end surface (5) of the clamping sleeve (7).

2. Apparatus according to claim 1, characterized in that on the upper side of the central part (21) at least one upward rib-shaped projection is provided.

3. Apparatus according to claim 1, characterized in that on the upper side of the central part (21) at least one recess shaped as a groove (20) is provided.

4. Apparatus according to one of claims 1 to 3, characterized in that the edge portion (28) of the plate (1) is directed against the main flow channel (6) of the housing (17).

5. Apparatus according to one of claims 1 to 4, characterized in that at the end surface (5) of the clamping sleeve (7) and/or at the annular shoulder (4) of the housing (17) circumjacent retention ribs and grooves (8, 9) for the plate (1) are formed.

6. Apparatus according to one of claims 1 to 5, characterized in that the clamping sleeve (7) is connected to a cap (11) to which a hinged cover (12) is attached and which is secured against axial removal from the cylindrical wall (24) of the housing (17).

## Revendications

1. Appareil médical pour l'injection ou le prélèvement de liquides à l'aide d'une seringue qui comporte un carter (17) à paroi cylindrique (24) dans lequel est engagé un fourreau de blocage (7) dont le passage (10) sert d'ouverture d'emmanchement pour l'appendice conique (15) de la seringue (14) et dont la surface frontale plane (5) applique la partie de bord (28) d'un corps de soupape agencé sous la forme d'une plaque élastique (1) comportant un orifice de passage de liquide auto-obturable contre un épaulement annulaire (4) du carter (17) dans la zone de transition avec un canal principal de passage d'écoulement (6), caractérisé en ce que la partie de bord (28) de la plaque (1) est reliée par l'intermédiaire de voiles radiaux élastiques (22) à une partie centrale (21) de la plaque (1), qui s'applique dans la condition de montage de la plaque (1) dans le plan de la partie de bord (28), contre la surface frontale (5) du fourreau de blocage (7).

2. Appareil selon la revendication 1, caractérisé en ce qu'il est prévu sur le côté supérieur de la partie centrale (21) au moins une saillie en forme de nervure dirigée vers le haut.

3. Appareil selon la revendication 1, caractérisé en ce qu'il est prévu sur le côté supérieur de la partie centrale (21) au moins un évidement en forme de rainure (20).

4. Appareil selon l'une quelconque des revendications 1 à 3, caractérisé en ce que la partie de bord (28) de la plaque (1) est dirigée vers le canal principal de passage d'écoulement (6) du carter (17).

5. Appareil selon l'une quelconque des revendications 1 à 4, caractérisé en ce que des nervures et des rainures de retenue (8, 9) de la plaque (1) sont formées sur la surface frontale (5) du fourreau de blocage (7) et/ou sur l'épaulement annulaire (4) du carter (17).

6. Appareil selon l'une quelconque des revendications 1 à 5, caractérisé en ce que le fourreau de blocage (7) est relié à un chapeau (11) sur lequel est fixé un couvercle rabattable (12) et qui est empêché d'être retiré axialement de la paroi cylindrique (24) du carter (17).

**Fig. 1**

**Fig. 2**

# Fig. 3

# Fig. 4